# EUROPEAN PATENT SPECIFICATION

(11) **EP 3 539 493 B1**
(45) Date of publication and mention of the grant of the patent: **03.01.2024**
(21) Application number: 19170497.2
(22) Date of filing: 15.02.2016
(51) Int. Cl.: A61B 17/80, A61F 2/44

(54) **EXPANDABLE OPEN WEDGE IMPLANT**
EXPANDIERBARES OPEN-WEDGE-IMPLANTAT
IMPLANT À COIN OUVERT EXTENSIBLE

(30) Priority: 14.02.2015 US 201562116385 P; 11.02.2016 US 201615041533; 11.02.2016 WO PCT/US2016/017505
(43) Date of publication of application: 18.09.2019
(62) Divisional of application: 16155755.8
(73) Proprietor: In2Bones USA, LLC, Memphis, TN 38119 (US)
(72) Inventor: WAHL, Rebecca Hawkins, Escondido, CA 92025 (US)
(74) Representative: Dehns

(56) References cited:
- EP-A1- 2 719 360
- US-A1- 2013 158 663
- US-A1- 2014 243 982
- US-A1- 2015 032 220

## Description

### FIELD

The field of the present disclosure generally relates to surgical implants. More particularly, the field of the invention relates to an apparatus for an expandable open wedge implant for performing open wedge osteotomies.

### BACKGROUND

An osteotomy is a surgical operation whereby a bone is cut to shorten, lengthen, or change its alignment. Osteotomy is sometimes performed to correct a hallux valgus (i.e., a bunion), or to straighten a bone that has healed crookedly following a fracture. Osteotomy is typically used to relieve pain in arthritis, especially of the hip and knee, as well as the feet. For instance, osteotomy may be used to correct conditions affecting the hip, such as a coxa vara, where the angle between the head and the shaft of the femur is reduced to less than 120 degrees. Osteotomy may also be used to correct conditions affecting the knee, such as a genu valgum, a condition in which the knees angle in and touch one another, and a genu varum, a condition characterized by an outward bowing of the lower legs in relation to the thighs. Osteotomy is also utilized in the anatomical areas of the spine and wrist. Further, various conditions affecting the feet may be treated by way of osteotomy, such as an Evans Procedure for lateral column lengthening, a Cotton Procedure for medial cuneiform opening wedge osteotomy in the foot, and the like.

Generally, there are two ways in which osteotomy is utilized to adjust the orientation of a bone, such as the tibia: 1) a closed wedge osteotomy; and 2) an open wedge osteotomy. With closed wedge osteotomies, a wedge of bone typically is removed from a bone and then the bone is manipulated so as to close the resulting gap, thereby re-orienting the bone relative to other bones and hence adjusting the manner in which load is transferred onto the bone. In the case of open wedge osteotomies, a cut is made into the bone being adjusted and then the bone is manipulated so as to open a wedge-like opening in the bone, whereby the bone is re-oriented relative to the other bones to adjust the manner in which load is transferred onto the bone. The bone is then secured with a desired wedge-like opening by way of screwing metal plates to the bone, or by way of inserting a wedge-shaped implant into the opening in the bone.

Although both closed wedge osteotomies and open wedge osteotomies are well known to provide substantial benefits to patients, such surgeries are procedurally challenging for surgeons. As will be appreciated, in the case of open wedge osteotomies, properly stabilizing the bone with the desired wedge-like opening during healing can be very difficult. Further, the wedge-shaped implants used in open wedge osteotomies generally must be matched to the size of the patient's anatomy and the degree of correction desired. The surgeon is, therefore, challenged with selecting a perfectly sized implant in which performing minor adjustments may be difficult. Further still, wedge-shaped implants used in open wedge osteotomies generally are procedure-specific. For example, an antero-medial procedure may require one configuration of an implant, while a lateral procedure may require another configuration of the implant, and the like. The surgeon is thus again challenged with selecting a perfectly-sized implant, as mentioned above.

The present disclosure is, therefore, generally directed to open wedge osteotomy procedures applied to feet, such as the Evans Procedure for lateral column lengthening and the Cotton Procedure for medial cuneiform opening wedge osteotomy, as well as osteotomies for other bone joint locations, and is intended to provide a new and improved osteotomy implant which addresses the foregoing issues.

EP2719360 relates to an interbody spinal implant system.

### SUMMARY

The present invention provides an open wedge implant for open wedge osteotomies, as presented in claim1. Preferred embodiments are set forth in the dependent claims. The open wedge implant comprises a wedge body including a first expandable portion and a second expandable portion connected together by way of an intervening distal attachment. In some embodiments, the first and second expandable portions may comprise roughened exterior surfaces that are configured for contacting bone portions exposed during an osteotomy. In some embodiments, the first and second expandable portions may comprise surfaces with apertures that are configured for contacting bone portions exposed during an osteotomy and allowing new bone to grow through. An expander is configured to separate the first and second expandable portions so as to increase a proximal thickness and a wedge angle of the wedge body. A proximal screw is configured to move the expander within the wedge body. Tightening the proximal screw draws the expander distally into the wedge body, thereby changing the open wedge implant from an initial, narrow wedge angle to an expanded wedge angle. In some embodiments, the open wedge implant is adapted for use in osteotomies performed in the feet, such as an Evans Procedure for lateral column lengthening, a Cotton Procedure for medial cuneiform opening wedge osteotomy, and the like.

In an embodiment, an open wedge implant for open wedge osteotomies comprises a wedge body comprising a first expandable portion and a second expandable portion connected together by way of an intervening distal attachment; an expander configured to separate the first and second expandable portions so as to increase a proximal thickness and a wedge angle of the wedge body; and a proximal screw configured to move the expander within the wedge body. In another embodiment, the open wedge implant is adapted for use in osteotomies performed in the feet, such as an Evans Procedure for lateral column lengthening, a Cotton Procedure for medial cuneiform opening wedge osteotomy, and the like.

In another embodiment, the first and second expandable portions respectively comprise a first face and a second face comprising roughened exterior surfaces of the open wedge implant that are configured for contacting bone portions exposed during an osteotomy. In another embodiment, the first and second expandable portions respectively comprise a first face and a second face comprising surfaces with apertures of the open wedge implant that are configured for contacting bone portions exposed during an osteotomy and allowing new bone to grow through. In another embodiment, the first and second expandable portions, and the distal attachment comprise a single component of material. In another embodiment, the first and second expandable portions, and the distal attachment comprise separate components that are coupled together by any one of various suitable fastening techniques.

In another embodiment, the wedge body is comprised of a semi-flexible material, such as polyetheretherketone (PEEK), or polyetherketoneketone (PEKK). In another embodiment, the wedge body is comprised of a metal alloy exhibiting shape memory and superelastic properties, such as Nitinol. In another embodiment, tightening the proximal screw draws the expander distally into the wedge body, thereby changing the open wedge implant from an initial configuration to an expanded configuration. In another embodiment, the initial configuration is characterized by a relatively narrow proximal thickness and a relatively small wedge angle of the open wedge implant. In another embodiment, the expanded configuration is characterized by a relatively larger value of the proximal thickness, the wedge angle being proportional to the proximal thickness. In another embodiment, the distal attachment biases the wedge body in the initial configuration, thereby maintaining an assembled state of the open wedge implant.

In another embodiment, the wedge body comprises a proximal opening configured to receive the expander, such that the first and second expandable portions increasingly separate as the expander is drawn distally into the proximal opening. In another embodiment, the proximal opening comprises a first bevel in the first expandable portion and a second bevel in the second expandable portion, the first and second bevels being configured to respectively contact a first taper and a second taper respectively disposed on top and bottom sides of the expander. In another embodiment, the first and second tapers give the expander a distally tapering thickness suitable for separating the first and second expandable portions.

In another embodiment, the expander comprises a recess configured to loosely retain a smooth portion of the proximal screw, the recess allowing free rotation of the smooth portion while a threaded portion of the proximal screw is rotatably engaged within a threaded channel disposed between the first and second expandable portions. In another embodiment, the expander comprises a countersink configured to retain a head portion of the proximal screw, allowing free rotation of the screw and preventing the expander from becoming disengaged from the screw, the countersink comprising a depth such that the head portion is positioned substantially entirely within the body of the expander and remains substantially flush with the proximal end of the wedge body. In another embodiment, the proximal screw comprises a proximal socket configured to facilitate engaging and rotating the proximal screw by way of a suitable tool, such as any one of a variety of appropriate drivers.

A method (which is not claimed) for a wedge implant for open wedge osteotomies comprises connecting a first expandable portion and a second expandable portion to an intervening distal attachment so as form a wedge body; configuring an expander to separate the first and second expandable portions so as to increase a proximal thickness and a wedge angle of the wedge body; and moving the expander within the wedge body by way of a proximal screw.

In another exemplary embodiment, moving the expander further comprises tightening the proximal screw so as to draw the expander distally into the wedge body, thereby separating the first and second expandable portions, and increasing the proximal thickness and the wedge angle of the wedge implant. In another exemplary embodiment, tightening further comprises engaging a proximal socket of the proximal screw and rotating the proximal screw by way of a suitable tool.

### BRIEF DESCRIPTION OF THE DRAWINGS

The drawings refer to embodiments of the present disclosure in which:
Figure 1 illustrates a perspective view of an embodiment of an expandable open wedge implant for open wedge osteotomies, according to the present disclosure;
Figure 2 illustrates a side plan view of an embodiment of an expandable open wedge implant for open wedge osteotomies in accordance with the present disclosure;
Figure 3 illustrates an exploded view of the embodiment of the expandable open wedge implant illustrated in Figs. 1-2;
Figure 4 illustrates a transverse cross-sectional view of the embodiment of the expandable open wedge implant illustrated in Figs. 1-2 in an initial configuration, according the present disclosure;
Figure 5 illustrates a transverse cross-sectional view of the embodiment of the expandable open wedge implant illustrated in Fig. 3 in an expanded configuration in accordance with the present disclosure;
Figure 6 illustrates a sagittal cross-sectional view of the embodiment of the expandable open wedge implant illustrated in Fig. 4 in the initial configuration in accordance with the present disclosure;
Figure 7 illustrates a sagittal cross-sectional view of the embodiment of the expandable open wedge implant illustrated in Fig. 5 in the expanded configuration, according the present disclosure;
Figure 8 illustrates a perspective view of an embodiment of an expandable open wedge implant for open wedge osteotomies, according to the present disclosure;
Figure 9 illustrates a side plan view of the expandable open wedge implant for open wedge osteotomies illustrated in Fig. 8, according to the present disclosure;
Figure 10 illustrates a perspective view of an embodiment of an expandable open wedge implant for open wedge osteotomies, according to the present disclosure; and
Figure 11 illustrates a top plan view of the expandable open wedge implant for open wedge osteotomies illustrated in Fig. 10, according to the present disclosure.

While the present disclosure is subject to various modifications and alternative forms, specific embodiments thereof have been shown by way of example in the drawings and will herein be described in detail. The invention should be understood to not be limited to the particular forms disclosed, but on the contrary, the intention is to cover all modifications, equivalents, and alternatives falling within the scope of the present disclosure.

### DETAILED DESCRIPTION

In the following description, numerous specific details are set forth in order to provide a thorough understanding of the present disclosure. It will be apparent, however, to one of ordinary skill in the art that the invention disclosed herein may be practiced without these specific details. In other instances, specific numeric references such as "first implant," may be made. However, the specific numeric reference should not be interpreted as a literal sequential order but rather interpreted that the "first implant" is different than a "second implant." Thus, the specific details set forth are merely exemplary. The specific details may be varied from and still be contemplated to be within the scope of the present disclosure. The term "coupled" is defined as meaning connected either directly to the component or indirectly to the component through another component. Further, as used herein, the terms "about," "approximately," or "substantially" for any numerical values or ranges indicate a suitable dimensional tolerance that allows the part or collection of components to function for its intended purpose as described herein.

In general, the present disclosure describes an apparatus for an open wedge implant for open wedge osteotomies. The open wedge implant comprises a wedge body including a first expandable portion and a second expandable portion connected together by way of an intervening distal attachment. The first and second expandable portions respectively comprise a first face and a second face comprising exterior surfaces that are configured for contacting bone portions exposed during an osteotomy. An expander is configured to separate the first and second expandable portions so as to increase a proximal thickness and a wedge angle of the wedge body. A proximal screw is configured to move the expander within the wedge body. Turning the proximal screw clockwise draws the expander distally into the wedge body, thereby changing the open wedge implant from an initial, narrow wedge angle to an expanded wedge angle. The distal attachment biases the wedge body toward the narrow wedge angle, thereby maintaining an assembled state of the open wedge implant. In some embodiments, the open wedge implant is adapted for use in osteotomies performed on the feet, such as an Evans Procedure for lateral column lengthening, a Cotton Procedure for medial cuneiform opening wedge osteotomy, and the like.

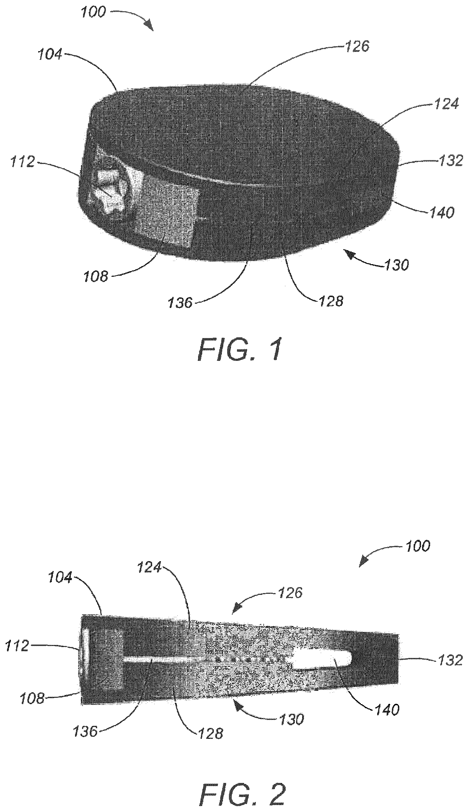

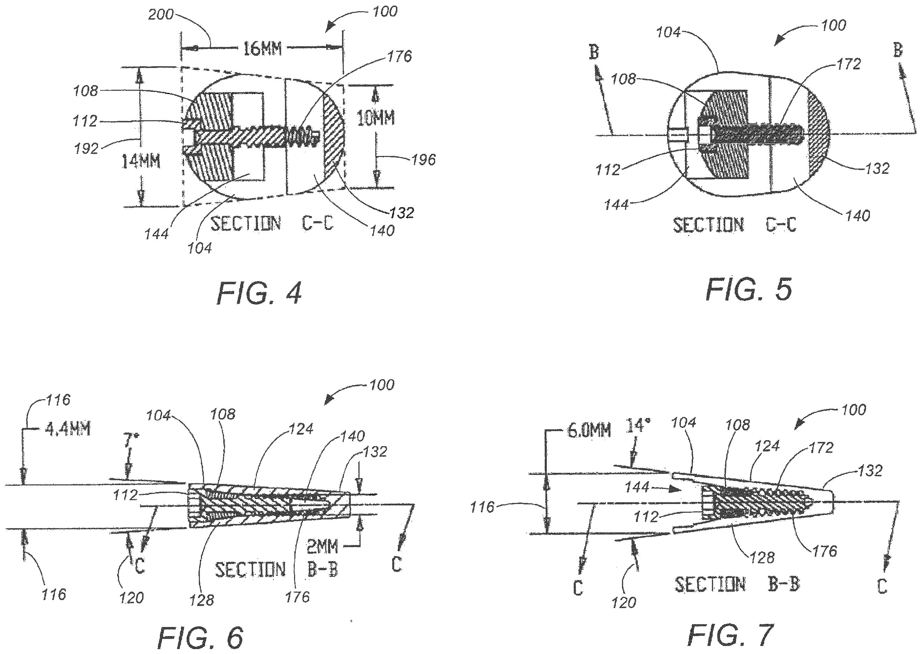

Figures 1-3 illustrate an embodiment of an expandable open wedge implant 100 for open wedge osteotomies in accordance with the present disclosure. The open wedge implant 100 comprises a wedge body 104, an expander 108, and a proximal screw 112. Broadly, turning the proximal screw 112 clockwise, by way of a suitable tool, draws the expander 108 into the wedge body 104, thereby changing the open wedge implant 100 from an initial configuration, as shown in Figs. 4 and 6, to an expanded configuration shown in Figs. 5 and 7. As described herein, the open wedge implant 100 is characterized by a relatively narrow proximal thickness 116 in the initial configuration illustrated in Fig. 6. In the expanded configuration, however, the proximal thickness 116 assumes a relatively larger value. As will be appreciated, a wedge angle 120 of the open wedge implant 100 is proportional to the proximal thickness 116. Thus, in the initial configuration, the wedge angle 120 assumes a small angle, whereas in the expanded configuration the wedge angle 120 possesses a relatively larger angle. It should be understood, therefore, that the wedge angle 120 of the open wedge implant 100 is adjustable by way of turning the proximal screw 112.

As illustrated in Figs. 1 and 2, the wedge body 104 comprises a first expandable portion 124 and a second expandable portion 128 connected together by way of an intervening distal attachment 132, such that the first and second expandable portions 124, 128 are separated by a gap 136. The first and second expandable portions 124, 128 respectively comprise a first face 126 and a second face 130. The first and second faces 126, 130 comprise exterior surfaces of the open wedge implant 100 that are configured for contacting bone portions exposed during an osteotomy. As will be recognized, the gap 136 allows for movement of the first and second expandable portions 124, 128 relatively to one another during adjustment of the open wedge implant 100. Further, the distal attachment 132 biases the wedge body 104 in the initial configuration, illustrated in Fig. 1, thereby maintaining an assembled state of the open wedge implant 100. A distal channel 140 facilitates deflection of the first and second expandable portions 124, 128, and operates to maintain a uniform value of the wedge angle 120 along a longitudinal dimension of the open wedge implant 100.

In the embodiment illustrated in Figs. 1 and 2, the first and second expandable portions 124, 128, and the distal attachment 132 comprise a single component of material. In some embodiments, however, the first and second expandable portions 124, 128, and the distal attachment 132 may be separate components that are coupled together by any of various suitable fastening techniques. The wedge body 104 preferably is comprised of a semi-flexible material, such as by way of non-limiting example, a thermoplastic polymer, such as polyetheretherketone (PEEK) and polyetherketoneketone (PEKK), or a metal alloy exhibiting elastic properties, such as Nitinol. In some embodiments, the expander 108 is comprised of the same material as the wedge body 104. In some embodiments, the expander 108 is comprised of a material that is more rigid than the material comprising the wedge body 104.

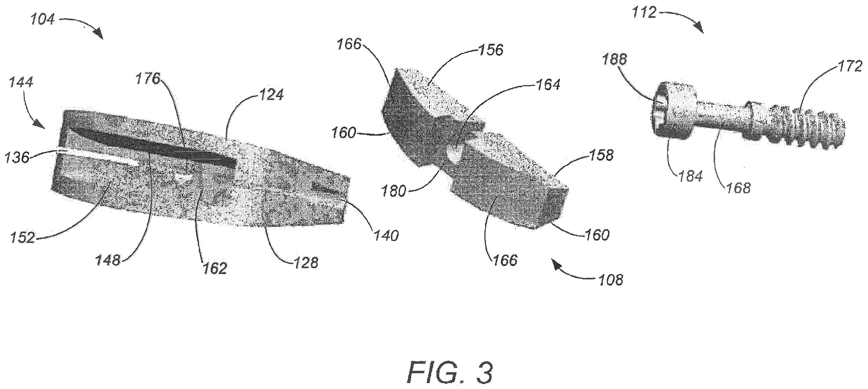

Figure 3 illustrates an exploded view of the wedge body 104, the expander 108, and the proximal screw 112 comprising the open wedge implant 100 illustrated in Figs. 1-2. The wedge body 104 comprises a proximal opening 144 configured to receive the expander 108, such that the first and second expandable portions 124, 128 increasingly separate as the expander 108 is drawn into the proximal opening 144. As such, the proximal opening 144 comprises a first bevel 148 in the first expandable portion 124, and a second bevel 152 in the second expandable portion 128. The first and second bevels 148, 152 are configured to respectively contact a first taper 156 and a second taper 160 respectively disposed on top and bottom sides of the expander 108. As illustrated in Fig. 3, the first and second tapers 156, 160 give the expander 108 a distally tapering thickness suitable for separating the first and second expandable portions 124, 128. A flat distal end 158 of the expander 108 is configured to contact a distal surface 162 of the proximal opening 144 when the open wedge implant 100 is placed into the expanded configuration illustrated in Fig. 5. A rounded proximal surface 166 of the expander 108 comprises a curvature which is substantially identical to a curvature of a proximal end of the wedge body 104 so as to give the open wedge implant 100 an exterior finish.

The expander 108 comprises a recess 164 configured to loosely retain a smooth portion 168 of the proximal screw 112. The recess 164 allows free rotation of the smooth portion 168 while a threaded portion 172 of the proximal screw 112 is rotatably engaged within a threaded channel 176 disposed between the first and second expandable portions 124, 128. The expander 108 comprises a countersink 180 configured to retain a head portion 184 of the proximal screw 112, thereby allowing free rotation of the screw while preventing the expander 108 from becoming disengaged from the screw. The countersink 180 further comprises a depth whereby the head portion 184 is positioned substantially entirely within the body of the expander 108. In the embodiment illustrated in Figs. 1-3, the depth of the countersink 180 is such that the head portion 184 remains substantially flush with the proximal end of the wedge body 104. A proximal socket 188 facilitates engaging and rotating the proximal screw 112 by way of a suitable tool, such as any of a variety of appropriate drivers.

It should be appreciated that the open wedge implant 100 may be advantageously used in various osteotomies performed in various locations within a patient's body. As such, the open wedge implant 100 may be implemented with a wide variety of shapes, sizes, and dimensions. The embodiment illustrated in Figs. 4-7 is adapted for use in osteotomies performed in the feet, such as by way of non-limiting example, an Evans Procedure for lateral column lengthening, a Cotton Procedure for medial cuneiform opening wedge osteotomy, and the like. Accordingly, the open wedge implant 100 illustrated in Figs. 4-7 is implemented with a shape and dimensions advantageously selected for osteotomies performed in the feet. It should be understood, however, that the open wedge implant 100 is not limited to osteotomies performed in the feet, nor is the open wedge implant 100 to be limited to the shape and dimensions illustrated in Figs. 4-7. Rather, it is to be understood that the open wedge implant 100 may be practiced with a variety of shapes and dimensions selected to advantageously accommodate osteotomies performed in other bone or bone-joint locations throughout the patient's body.

Referring specifically to Figs, 4-7, the open wedge implant 100 generally comprises an initially trapezoidal transverse cross-section with parallel proximal and distal side dimensions 192, 196, wherein the corners of the trapezoid are rounded to form the shape of the open wedge implant 100 illustrated in Fig. 1. In the illustrated embodiment of Fig. 4, the proximal side 192 is substantially 14 millimeters (mm) and the distal side 196 is substantially 10 mm, with a longitudinal dimension 200 being substantially 16 mm. It will be appreciated that implementing the open wedge implant 100 with the proximal side 192 being wider than the distal side 196 gives the implant a slightly wedge-shaped transverse cross-section. It will be further appreciated that the slightly wedge-shaped transverse cross-section advantageously facilitates inserting the open wedge implant 100 into an opening in bone formed during an osteotomy.

As discussed above, tightening the proximal screw 112 draws the expander 108 into the proximal opening 144, and thus changes the open wedge implant 100 from the initial configuration, shown in Figs. 4 and 6, to an expanded configuration wherein the first and second expandable portions 124, 128 are deflected away from one another. The open wedge implant 100 is placed into a fully expanded configuration once the distal end 158 of the expander 108 contacts the distal surface 162, as illustrated in Fig 5. In the fully expanded configuration, the first and second expandable portions 124, 128 are positioned at a maximal separation from one another, thereby maximizing the proximal thickness 116 and the wedge angle 120 of the open wedge implant 100. In the initial configuration of the open wedge implant 100, illustrated in Figs. 6-7, the proximal thickness is substantially 4.4 mm and the wedge angle 120 is substantially 7 degrees. In the fully expanded configuration, wherein the proximal screw 112 has been tightened until the distal end 158 contacts the distal surface 166, as illustrated in Figs. 5 and 7, the proximal thickness is substantially 6.0 mm and the wedge angle 120 is substantially 14 degrees. As will be appreciated, throughout the expandable range of the first and second expandable portions 124, 128, the thickness of the distal attachment 132 remains essentially unchanged. In the illustrated embodiment of Figs. 6-7, the thickness of the distal attachment 132 is substantially 2 mm. As mentioned above, however, various shapes and dimensions may be incorporated into various embodiments of the open wedge implant 100 so as to utilize the open wedge implant in osteotomies performed in various bone or bone-joint locations within the patient's body without limitation.

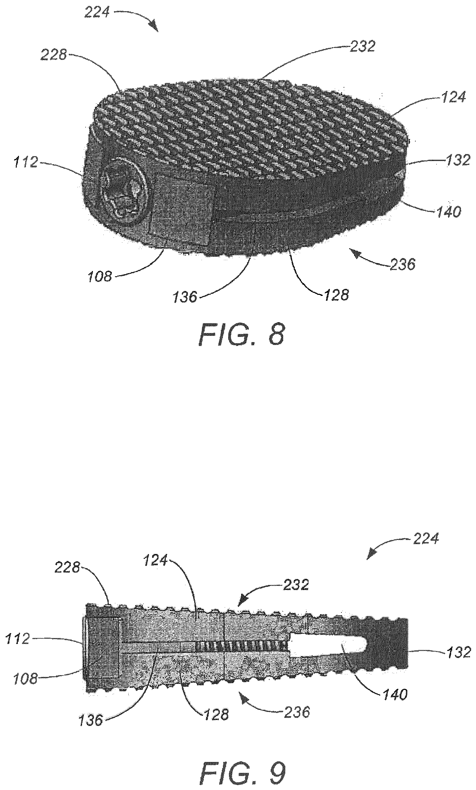

Figures 8-9 illustrate an embodiment of an expandable open wedge implant 224 for open wedge osteotomies, according to the present disclosure. The open wedge implant 224 is substantially similar to the open wedge implant 100 illustrated in Figs. 1-2, with the exception that the open wedge implant 224 comprises a wedge body 228 including a first roughened face 232 and a second roughened face 236. In some embodiments, the first and second roughened faces 232, 236 comprise any of various surface features that are machined into the first and second faces 126, 130. In some embodiments, the first and second roughened faces 232, 236 comprise a roughened surface coating, such as by way of non-limiting example, a Titanium plasma spray coating, or other similar material, applied to a PEEK or PEKK substrate. It will be appreciated that the first and second roughened faces 232, 236 provide a relatively more effective contact between the open wedge implant 224 and bone portions exposed during an osteotomy.

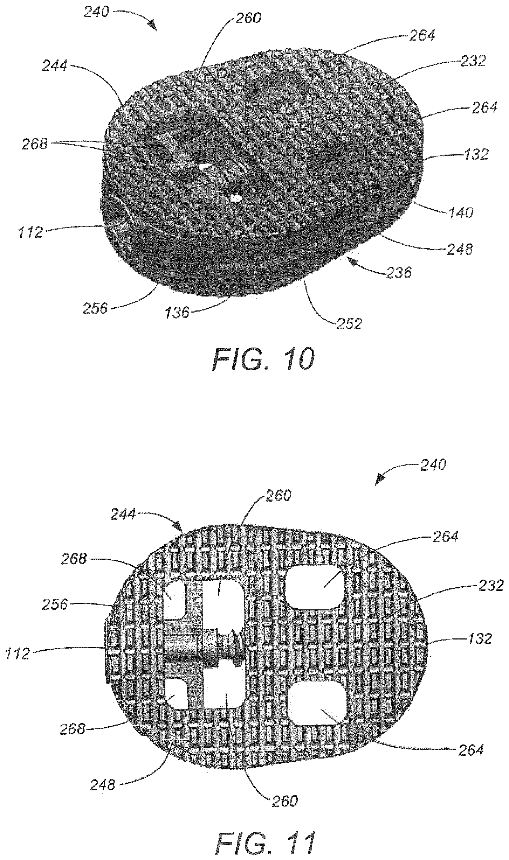

Figures 10-11 illustrate an embodiment of an expandable open wedge implant 240 configured for open wedge osteotomies in accordance with the present disclosure. The open wedge implant 240 is substantially similar to the open wedge implant 224 illustrated in Figs. 8-9, with the exception that the open wedge implant 240 comprises a wedge body 244 which includes a first expandable portion 248 and a second expandable portion 252, and an expander 256. In the embodiment illustrated in Figs. 10-11, each of the first and second expandable portions 248, 252 comprises a proximal window 260 and a pair of distal windows 264. As best shown in Fig. 11, the windows 260, 264 in the first and second expandable portions 248, 252 are vertically aligned so as to form holes passing through the open wedge implant 240. It will be appreciated that the holes operate to promote bone formation in the area of the osteotomy, and form pathways for new bone growth passing through the open wedge implant 240. Accordingly, the expander 256 comprises openings 268 so as to minimize any obstruction of the proximal window 260 once the expander 256 has been drawn distally into the opening 144, as described herein. It should be understood that the open wedge implant 240 is not to be limited to the number and shapes of the windows and openings illustrated in Figs. 10-11, but rather any number and shape of the windows and openings may be incorporated into the open wedge implant 240 without limitation.

It is envisioned that open wedge implants, according to the present disclosure, are to be suitably sterilized for surgeries, and packaged into sterilized containers. In some embodiments, the wedge body 104 is packaged into a first sterile container, the expander 108 is packaged into a second sterile container, and the proximal screw 112 is packaged into a third sterile container. The first, second, and third sterile containers are then bundled together into a single, exterior container, thereby forming a convenient surgery-specific osteotomy wedge implant package. In some embodiments, the open wedge implant 100 is assembled and packaged into a single sterile container, thereby removing a need for assembly before or during surgery. It is envisioned that other packaging techniques will be apparent to those skilled in the art without deviating from the scope of the present disclosure.

## Claims

1. An open wedge implant (100) for open wedge osteotomies, comprising:
a wedge body (104) comprising a thermoplastic polymer material and a first expandable portion (124) and a second expandable portion (128) connected together by way of an intervening distal attachment (132), the wedge body configured such that a cross-section at a proximal end (116) is larger than at a distal end and a proximal opening (144);
the first and second expandable portions respectively comprise a first face (126) and a second face (130) comprising roughened exterior surfaces, the roughened exterior surfaces spanning the entire first and second faces configured for contacting bone portions exposed during osteotomy, such that the roughened exterior surfaces comprise a titanium plasma spray coating applied to a polyetheretherketone (PEEK), or polyetherketoneketone (PEKK) substrate;
an expander (108) configured to separate the first and second expandable portions (124, 128) so as to increase a proximal thickness and a wedge angle of the wedge body (104), the distal end (158) comprising a flat portion configured to contact a distal surface (162) of the proximal opening (144), wherein the proximal opening comprises:
a first bevel (148) in the first expandable portion and a second bevel (152) in the second expandable portion which are configured to contact a first taper (156) and a second taper (160) in the expander;
wherein a rounded proximal surface (166) of the expander comprises a curvature which is identical to a curvature of the proximal end of the wedge body;
a distal channel (140) configured to facilitate deflection of the first and second expandable portions, such that a uniform wedge angle is maintained along a longitudinal dimension of the wedge implant;
a proximal screw (112) configured to move the expander (108) within the wedge body (104), whereby a wedge angle of the open wedge implant is adjustable by way of turning the proximal screw.

2. The open wedge implant of claim 1, wherein the open wedge implant (100) is adapted for use in osteotomies performed in the feet.

3. The open wedge implant of claim 1, wherein the first and second expandable portions (124, 128), and the distal attachment comprise a single component of material.

4. The open wedge implant of claim 1, wherein the first and second expandable portions (124, 128), and the distal attachment comprise separate components that are coupled together by any one of various suitable fastening techniques.

5. The open wedge implant of any preceding claim, wherein the wedge body (104) is comprised of a semi-flexible material, such as polyetheretherketone (PEEK), or polyetherketoneketone (PEKK).

6. The open wedge implant of claim 1, wherein tightening the proximal screw (112) draws the expander (108) distally into the wedge body, thereby changing the open wedge implant from an initial configuration to an expanded configuration.

7. The open wedge implant of claim 6, wherein the initial configuration is **characterized by** a relatively narrow proximal thickness and a relatively small wedge angle of the open wedge implant (100), or wherein the expanded configuration is **characterized by** a relatively larger value of the proximal thickness, the wedge angle being proportional to the proximal thickness.

8. The open wedge implant of claim 6, wherein the distal attachment biases the wedge body (104) in the initial configuration, thereby maintaining an assembled state of the open wedge implant (100).

9. The open wedge implant of claim 1, wherein the proximal opening is configured to receive the expander, such that the first and second expandable portions increasingly separate as the expander is drawn distally into the proximal opening, and preferably wherein the first and second tapers give the expander a distally tapering thickness suitable for separating the first and second expandable portions (124, 128).

10. The open wedge implant of claim 1, wherein the expander (108) comprises a recess configured to loosely retain a smooth portion of the proximal screw (112), the recess allowing free rotation of the smooth portion while a threaded portion of the proximal screw is rotatably engaged within a threaded channel disposed between the first and second expandable portions (124, 128).

11. The open wedge implant of claim 1, wherein the expander (108) comprises a countersink configured to retain a head portion of the proximal screw (112), allowing free rotation of the screw and preventing the expander (108) from becoming disengaged from the screw, the countersink comprising a depth such that the head portion is positioned substantially entirely within the body of the expander (108) and remains substantially flush with the proximal end of the wedge body.

12. The open wedge implant of claim 1, wherein the proximal screw (112) comprises a proximal socket configured to facilitate engaging and rotating the proximal screw by way of a suitable tool, such as any one of a variety of appropriate drivers.

## Patentansprüche

1. Open-Wedge-Implantat (100) für Open-Wedge-Osteotomien, umfassend:
einen Keilkörper (140), der ein thermoplastisches Polymermaterial und einen ersten ausdehnbaren Abschnitt (124) und einen zweiten ausdehnbaren Abschnitt (128) umfasst, die über eine dazwischenliegende distale Befestigung miteinander verbunden sind, wobei der Keilkörper so konfiguriert ist, dass ein Querschnitt an einem proximalen Ende (116) größer ist als an einem distalen Ende und einer proximalen Öffnung (144);
wobei der erste und der zweite ausdehnbare Abschnitt jeweils eine erste Stirnseite (126) und eine zweite Stirnseite (130) umfassen, die angeraute Außenoberflächen umfassen, wobei die angerauten Außenoberflächen, die die gesamte erste und zweite Stirnseiten überspannen, konfiguriert sind, um Knochenabschnitte zu kontaktieren, die während einer Osteotomie freiliegen, sodass die angerauten Außenoberflächen eine Titanplasmaspraybeschichtung umfassen, die auf ein Polyetheretherketon- (PEEK) oder Polyetherketoneketon-(PEKK)-Substrate aufgetragen ist;
einen Expander (108), der konfiguriert ist, um den ersten und den zweiten ausdehnbaren Abschnitt (124, 128) zu trennen, um so eine proximale Dicke und einen Keilwinkel des Keilkörpers (104) zu erhöhen, wobei das distale Ende (158) einen planen Abschnitt umfasst, der konfiguriert ist, um eine distale Oberfläche (162) der proximalen Öffnung (144) zu kontaktieren, wobei die proximale Öffnung Folgendes umfasst:
eine erste Abschrägung (148) in dem ersten ausdehnbaren Abschnitt und eine zweite Abschrägung (152) in dem zweiten ausdehnbaren Abschnitt, die konfiguriert sind, um eine erste Verjüngung (156) und eine zweite Verjüngung (160) in dem Expander zu kontaktieren;
wobei eine abgerundete proximale Oberfläche (166) des Expanders eine Krümmung umfasst, die identisch ist zu einer Krümmung des proximalen Endes des Keilkörpers;
einen distalen Kanal (140), der konfiguriert ist, um Biegung des ersten und des zweiten ausdehnbaren Abschnitts so zu erleichtern, dass ein einheitlicher Keilwinkel entlang einer Längsabmessung des Keilimplantats aufrechterhalten wird;
eine proximale Schraube (112), die konfiguriert ist, um den Expander (108) innerhalb des Keilkörpers (104) zu bewegen, wodurch ein Keilwinkel des Open-Wedge-Implantats durch Drehen der proximalen Schraube justierbar ist.

2. Open-Wedge-Implantat nach Anspruch 1, wobei das Open-Wedge-Implantat (100) zur Verwendung bei an den Füßen durchgeführten Osteotomien angepasst ist.

3. Open-Wedge-Implantat nach Anspruch 1, wobei der erste und der zweite ausdehnbare Abschnitt (124, 128) und die distale Befestigung eine einzelne Materialkomponente umfassen.

4. Open-Wedge-Implantat nach Anspruch 1, wobei der erste und der zweite ausdehnbare Abschnitt (124, 128) und die distale Befestigung separate Komponenten umfassen, die durch eine von verschiedenen geeigneten Befestigungstechniken miteinander gekoppelt sind.

5. Open-Wedge-Implantat nach einem der vorstehenden Ansprüche, wobei der Keilkörper (104) aus einem halbflexiblen Material, wie etwa Polyetheretherketon (PEEK) oder Polyetherketonketon (PEKK), besteht.

6. Open-Wedge-Implantat nach Anspruch 1, wobei Anziehen der proximalen Schraube (112) den Expander (108) distal in den Keilkörper hineinzieht, wodurch das Open-Wedge-Implantat von einer anfänglichen Konfiguration zu einer ausgedehnten Konfiguration geändert wird.

7. Open-Wedge-Implantat nach Anspruch 6, wobei die anfängliche Konfiguration durch eine relativ schmale proximale Dicke und einen relativ schmalen Keilwinkel des Open-Wedge-Implantats (100) gekennzeichnet ist, oder wobei die ausgedehnte Konfiguration durch einen relativ größeren Wert der proximalen Dicke gekennzeichnet ist, wobei der Keilwinkel proportional zu der proximalen Dicke ist.

8. Open-Wedge-Implantat nach Anspruch 6, wobei die distale Befestigung den Keilkörper (104) in die anfängliche Konfiguration vorspannt, wodurch ein montierter Zustand des Open-Wedge-Implantats (100) aufrechterhalten wird.

9. Open-Wedge-Implantat nach Anspruch 1, wobei die proximale Öffnung konfiguriert ist, um den Expander so aufzunehmen, dass der erste und der zweite ausdehnbare Abschnitt sich zunehmend trennen, wenn der Expander distal in die proximale Öffnung hineingezogen wird, und wobei die erste und die zweite Verjüngung vorzugweise dem Expander eine sich distal verjüngende Dicke verleihen, die geeignet ist zum Trennen des ersten und des zweiten ausdehnbaren Abschnitts (124, 128).

10. Open-Wedge-Implantat nach Anspruch 1, wobei der Expander (108) eine Vertiefung umfasst, die konfiguriert ist, um einen glatten Abschnitt der proximalen Schraube (112) lose zu halten, wobei die Vertiefung eine freie Drehung des glatten Abschnitts erlaubt, während ein Gewindeabschnitt der proximalen Schraube drehbar in einem zwischen dem ersten und dem zweiten ausdehnbaren Abschnitt (124, 128) angeordneten Gewindekanal in Eingriff steht.

11. Open-Wedge-Implantat nach Anspruch 1, wobei der Expander (108) eine Versenkung umfasst, die konfiguriert ist, um einen Kopfabschnitt der proximalen Schraube (112) zu halten, was eine freie Drehung der Schraube erlaubt und verhindert, dass der Expander (108) von der Schraube gelöst wird, wobei die Versenkung eine Tiefe umfasst, sodass der Kopfabschnitt im Wesentlichen vollständig innerhalb des Körpers des Expanders (108) positioniert ist und im Wesentlichen bündig mit dem proximalen Ende des Keilkörpers bleibt.

12. Open-Wedge-Implantat nach Anspruch 1, wobei die proximale Schraube (112) eine proximale Buchse umfasst, die konfiguriert ist, um Eingreifen und Drehen der proximalen Schraube mittels eines geeigneten Werkzeugs, wie etwa einem von einer Vielzahl von geeigneten Drehern, zu erleichtern.

## Revendications

1. Implant à coin ouvert (100) pour des ostéotomies à coin ouvert, comprenant :
un corps de coin (104) comprenant un matériau polymère thermoplastique et une première portion extensible (124) et une seconde portion extensible (128) raccordées ensemble au moyen d'une fixation distale d'intervention (132), le corps de coin étant configuré de sorte qu'une section transversale au niveau d'une extrémité proximale (116) soit plus grande qu'au niveau d'une extrémité distale et d'une ouverture proximale (144) ;
les première et seconde portions extensibles comprennent respectivement une première face (126) et une seconde face (130) comprenant des surfaces extérieures rugosifiées, les surfaces extérieures rugosifiées s'étendant sur l'intégralité des première et secondes faces configurées pour venir au contact de portions osseuses exposées pendant une ostéotomie, de sorte que les surfaces extérieures rugosifiées comprennent un revêtement par pulvérisation de plasma de titane appliqué à un substrat en polyétheréthercétone (PEEK) ou en polyéthercétonecétone (PEKK) ;
un extenseur (108) configuré pour séparer les première et seconde portions extensibles (124, 128) de façon à augmenter une épaisseur proximale et un angle de coin du corps de coin (104), l'extrémité distale (158) comprenant une portion plate configurée pour venir au contact d'une surface distale (162) de l'ouverture proximale (144), dans lequel l'ouverture proximale comprend :
un premier biseau (148) dans la première portion extensible et un second biseau (152) dans la seconde portion extensible qui sont configurés pour venir au contact d'une première conicité (156) et d'une seconde conicité (160) dans l'extenseur ;
dans lequel une surface proximale arrondie (166) de l'extenseur comprend une courbure qui est identique à une courbure de l'extrémité proximale du corps de coin ;
un canal distal (140) configuré pour faciliter la déviation des première et seconde portions extensibles, de sorte qu'un angle de coin uniforme soit maintenu le long d'une dimension longitudinale de l'implant à coin ;
une vis proximale (112) configurée pour déplacer l'extenseur (108) au sein du corps de coin (104), selon lequel un angle de coin de l'implant à coin ouvert est réglable au moyen d'une rotation de la vis proximale.

2. Implant à coin ouvert selon la revendication 1, dans lequel l'implant à coin ouvert (100) est adapté pour une utilisation dans des ostéotomies réalisées dans les pieds.

3. Implant à coin ouvert selon la revendication 1, dans lequel les première et seconde portions extensibles (124, 128) et la fixation distale comprennent un composant de matériau unique.

4. Implant à coin ouvert selon la revendication 1, dans lequel les première et seconde portions extensibles (124, 128) et la fixation distale comprennent des composants séparés qui sont couplés ensemble par l'une quelconque de diverses techniques d'attache adéquates.

5. Implant à coin ouvert selon une quelconque revendication précédente, dans lequel le corps de coin (104) est composé d'un matériau semi-flexible, tel que le polyétheréthercétone (PEEK), ou le polyéthercétonecétone (PEKK).

6. Implant à coin ouvert selon la revendication 1, dans lequel un serrage de la vis proximale (112) tire l'extenseur (108) de façon distale dans le corps de coin, changeant ainsi l'implant à coin ouvert d'une configuration initiale à une configuration étendue.

7. implant à coin ouvert selon la revendication 6, dans lequel la configuration initiale est **caractérisée par** une épaisseur proximale relativement étroite et un angle de coin relativement petit de l'implant à coin ouvert (100), ou dans lequel la configuration étendue est **caractérisée par** une valeur relativement plus grande de l'épaisseur proximale, l'angle de coin étant proportionnel à l'épaisseur proximale.

8. Implant à coin ouvert selon la revendication 6, dans lequel la fixation distale sollicite le corps de coin (104) dans la configuration initiale, préservant ainsi un état assemblé de l'implant à coin ouvert (100).

9. Implant à coin ouvert selon la revendication 1, dans lequel l'ouverture proximale est configurée pour recevoir l'extenseur, de sorte que les première et seconde portions extensibles se séparent de plus en plus à mesure que l'extenseur est tiré distalement dans l'ouverture proximale, et de préférence dans lequel les première et seconde conicités donnent à l'extenseur une épaisseur s'effilant distalement convenant pour séparer les première et seconde portions extensibles (124, 128).

10. Implant à coin ouvert selon la revendication 1, dans lequel l'extenseur (108) comprend un évidement configuré pour retenir sans serrer une portion lisse de la vis proximale (112), l'évidement permettant une libre rotation de la portion lisse pendant qu'une portion filetée de la vis proximale est engagée en rotation au sein d'un canal fileté disposé entre les première et seconde portions extensibles (124, 128).

11. Implant à coin ouvert selon la revendication 1, dans lequel l'extenseur (108) comprend une fraise configurée pour retenir une portion de tête de la vis proximale (112), permettant une libre rotation de la vis et empêchant l'extenseur (108) de se désengager de la vis, la fraise comprenant une profondeur telle que la portion de tête est positionnée sensiblement dans son intégralité au sein du corps de l'extenseur (108) et reste sensiblement de niveau avec l'extrémité proximale du corps de coin.

12. Implant à coin ouvert selon la revendication 1, dans lequel la vis proximale (112) comprend une cuvette proximale configurée pour faciliter l'engagement et la rotation de la vis proximale à l'aide d'un outil adéquat, tel que l'un quelconque d'une variété d'organes d'entraînement appropriés.
